# FASCICULE DE BREVET EUROPEEN

(11) **EP 1 334 206 B1**
(45) Date de publication et mention de la délivrance du brevet: **24.05.2006**
(21) Numéro de dépôt: 01996622.5
(22) Date de dépôt: 16.11.2001
(51) Int. Cl.: C12Q 1/04, C12Q 1/44, C12Q 1/10, C12N 1/20, C12Q 1/34, C12Q 1/37, C12Q 1/42

(54) **PROCEDE ET MILIEU DE DETECTION/IDENTIFICATION DE MICROORGANISMES A ACTIVITE ESTERASE ET/OU OSIDASE ET/OU PEPTIDASE ET/OU SULFATASE ET/OU PHOSPHATATE**
VERFAHREN UND MEDIUM ZUM NACHWEISEN/IDENTIFIZIEREN VON MIKROORGANISMEN MIT ESTERASE- UND/ODER OSIDASE- UND/ODER PEPTIDASE- UND/ODER SULFATASE- UND/ODER PHOSPHATASEAKTIVITÄT
METHOD AND MEDIUM FOR DETECTING/IDENTIFYING MICRO-ORGANISMS WITH ESTERASE AND/OR OSIDASE AND/OR PEPTIDASE AND/OR SULPHATASE AND/OR PHOSPHATASE ACTIVITY

(30) Priorité: 17.11.2000 FR 0014879
(43) Date de publication de la demande: 13.08.2003
(73) Titulaire: BIO MERIEUX, 69280 Marcy l'Etoile (FR)
(72) Inventeur: GILBERT, Sandra, F-69009 LYON (FR); ROGER-DALBERT, Céline, F-01150 VAULX-EN-BUGEY (FR); ORENGA, Sylvain, F-01160 NEUVILLE SUR AIN (FR)
(74) Mandataire: Fleurance, Raphael
(86) Numéro de dépôt international: PCT/FR2001/003611
(87) Numéro de publication internationale: WO 2002/040706

(56) Documents cités:
- EP-A- 0 544 605
- WO-A-95/04157
- WO-A-99/41409
- FR-A- 2 697 028
- US-A- 4 717 658
- US-A- 4 927 927
- US-A- 4 994 376
- US-A- 5 296 370
- US-A- 5 786 167
- COOKE VENITIA M ET AL: "A novel chromogenic ester agar medium for detection of salmonellae." APPLIED AND ENVIRONMENTAL MICROBIOLOGY, vol. 65, no. 2, février 1999 (1999-02), pages 807-812, XP002177548 ISSN: 0099-2240
- MANAFI M: "New developments in chromogenic and fluorogenic culture media." INTERNATIONAL JOURNAL OF FOOD MICROBIOLOGY, vol. 60, no. 2-3, 2000, pages 205-218, XP002177549 ISSN: 0168-1605

## Description

Le domaine de l'invention est celui de l'analyse microbiologique par voie biochimique, et en particulier de la détection et de l'identification de souches bactériennes par ensemencement de milieux réactionnels, en particulier des milieux nutritifs. Ces derniers comprennent des substrats chromogènes ou fluorogènes susceptibles de réagir avec les enzymes spécifiques des souches recherchées, en produisant une coloration ou une fluorescence de chaque colonie concernée.

Dans le cadre de la présente invention, on s'intéresse plus particulièrement à la détection/identification de micro-organismes pathogènes ou indicateurs de qualité, que ce soit dans le milieu médical ou le milieu industriel, et plus particulièrement de microorganismes à activité enzymatique du type estérase, osidase, peptidase, sulfatase ou phosphatase, par exemple les bactéries ou levures des genres *Salmonella, Pseudomonas, Listeria, Staphylococcus, Enterococcus, Candida* et plus particulièrement encore des espèces *Escherichia coli, Listeria monocytogenes, Staphylococcus aureus, Candida albicans.*

Les souches d'*Escherichia coli* sont souvent mises en évidence par la révélation d'une activité enzymatique du type osidase telle que l'activité β-glucuronidase ou β-galactosidase.

De la même façon, le genre *Listeria* est détecté par la mise en évidence de l'activité β-glucosidase.

Une activité aminopeptidase peut également être utilisée pour révéler un groupe, un genre ou une espèce de micro-organismes. L'activité Alanine-aminopeptidase, par exemple, permet de différencier les bactéries à Gram négatif des bactéries à Gram positif.

Le genre *Salmonella,* responsable chez l'homme de diverses infections (fièvre typhoïde, intoxication alimentaire), possède des estérases non spécifiques capables d'hydrolyser des substrats synthétiques chromogènes, par exemple indigogéniques.

La détection/identification de salmonelles, et plus généralement de bactéries à activité estérase est classiquement réalisée sur des milieux gélosés ou liquides d'isolement, qui permettent la détection/identification des colonies suspectes de bactéries à activité estérase, notamment les salmonelles. L'ensemencement de tels milieux s'opère par trempage dudit milieu dans l'échantillon analysé ou par mise en contact de l'échantillon avec le milieu.

Les bactéries à activités estérases, osidases, peptidases, sulfatases ou encore phosphatases possèdent dans leur patrimoine enzymatique des estérases, osidases, peptidases, sulfatases ou phosphatases qui clivent les liaisons cibles des substrats présents dans le milieu et qui libèrent ainsi la partie chromophore ou fluorophore activée desdits substrats. Il en résulte une coloration ou une fluorescence qui révèle l'hydrolyse, et donc la présence de bactéries ou de colonies de bactéries cibles.

Pour pouvoir réaliser des tests de routine à grande envergure, il est nécessaire que les milieux de détection/identification soient stables et permettent de simplifier au maximum les procédés de détection/identification correspondant, en limitant les manipulations. En outre, il importe que les procédés offrent une haute sensibilité (intensité de coloration), ainsi qu'une spécificité de premier ordre. La vitesse de révélation des colonies suspectes est également un paramètre fondamental de ces types de milieux et procédés de détection/identification de bactéries présentant les activités enzymatiques susmentionnées.

Or, il est connu que les substrats d'enzymes telles que les estérases, les osidases, les peptidases, les sulfatases ou les phosphatases posent des problèmes de compatibilité avec les milieux de culture pour microorganismes et en particulier pour les bactéries possédant ces activités. De plus, de tels substrats ne sont pas stables dans le temps, ce qui induit une diminution de la sensibilité vis-à-vis de l'activité enzymatique concernée avec l'allongement du temps de conservation.

Dans ce contexte, on connaît au travers de l'article scientifique intitulé *"Synthèse de substrats indigogéniques. Mise en évidence de l'activité estérasique des salmonelles": A. Agban* et al., *Eur. J. Med. Chem. (1990) 25, 697-699,* des milieux de culture gélosés comprenant des substrats indigogéniques, à savoir notamment le Pélargonate (C9) de Bromo-5-indoxyle et un sel biliaire, à savoir le Déoxycholate de Sodium. De tels milieux de culture souffrent des mêmes inconvénients que ceux évoqués ci-dessous en référence au document FR-2 697 028.

Le brevet FR-2 697 028 divulgue un milieu de culture pour la mise en évidence de salmonelles comprenant un substrat d'estérase chromogène constitué par un ester de l'acide caprylique avec un reste indole (5-Bromo-4-chloro-3-indolyl-caprylate), ainsi qu'un détergent choisi parmi les sels biliaires (Désoxycholate de Sodium). Ce chromogène et ce sel biliaire sont contenus dans un milieu nutritif permettant la croissance des salmonelles. Selon l'enseignement du FR-2 697 028, le sel biliaire est ajouté directement au milieu sélectif dans lequel se trouve déjà inclus le substrat d'estérase.

Un autre inconvénient lié à l'emploi de sels biliaires tient au fait que ces derniers sont des matières premières d'origine animale, ce qui sous-tend une certaine variabilité de la qualité.

De plus, les résultats en termes d'activité biologique sont perfectibles.

Ce milieu de culture n'offre pas toutes les garanties souhaitables en termes de stabilité du substrat d'estérase. En outre, il s'avère que ce dernier n'est pas complètement miscible avec le milieu de culture. Il est clair que cela nuit à la qualité des résultats obtenus sur le plan de la sensibilité, de la rapidité et de la stabilité.

Il doit également être noté que le milieu de culture selon le FR-2 697 023 se présente sous forme de poudre. Cela oblige l'utilisateur à exécuter une opération de reconstitution préalable du milieu liquide ou gélifié. Cette contrainte résulte du manque de stabilité des substrats d'estérase mis en oeuvre. De plus, le milieu gélosé préparé selon l'enseignement du FR-2 697 028 n'est pas translucide. Ceci risque de compromettre la lecture des colorations associées aux éventuelles colonies de bactéries cibles.

La demande de brevet PCT WO-92/17607 concerne un milieu de détection pour salmonelles contenant du TERGITOL-4® (sulfate hydrogéné de 7-éthyl-2-méthyl-4-undécanonate ou son sel de sodium). Cet additif est sensé améliorer la sélectivité du milieu de détection vis-à-vis des salmonelles-cibles. La concentration en TERGITOL-4® peut varier de 2 à 30 ml/l dans le milieu de culture à base de gélose agar-xyloselysine. Selon ce document, la détection de salmonelles repose uniquement sur un principe de croissance sélective par compétition.

La demande PCT WO-99/41409 concerne des substrats d'estérase chromogènes pour la détection de salmonelles. Cette demande PCT correspond au document COOKE VENITIA M. ETAL; APPLIED AND ENVIRONMENTAL MICROBIOLOGY, vol. 65, n°2 Février 1999 (1990-02) pages 807-812.

Il est proposé selon ce document de mettre en oeuvre un composé chromogène qui réagit avec une estérase/lipase spécifique du genre *Salmonella* et ayant une affinité pour les esters d'acide gras en C8. Le composé chromogène dont il s'agit, comprend un anion et un cation de formule : [4-[2-(4-octanoyloxy-3,5-diméthoxyphényl)-vinyl]-quinolinium-1-(propan-3-yle) carboxylate]⁺, [bromure ou chlorure]. Plus précisément, les substrats mis en oeuvre sont des esters en C8 par exemple, du cation carboxylate.

En outre, le procédé selon le WO-99/41409 décrit la mise en oeuvre d'ester de Sorbitan et d'acides gras (en particulier : acide monolautique : TWEEN® 20). Ces produits sont employés à titre de détergent à raison de 2 g par litre de milieu.

En outre, le Substrat spécifique selon l'enseignement de cette demande PCT peut être introduit dans le milieu de culture nutritif en mélange avec du Méthanol, de l'Ethanol ou de la N,N-Diméthylformamide (DMF). Le détergent éventuel n'est pas introduit conjointement au substrat et au solvant, mais séparément.

En tout état de cause, les moyens divulgués dans le WO-99/41409 ne sont pas présentés comme procurant une amélioration de la stabilité des substrats enzymatiques contenus dans le milieu de détection.

On connaît également au travers de différents documents, des milieux de cultures comportant plusieurs substrats pour la détection d'une seule activité enzymatique, mais également des milieux de culture comportant plusieurs substrats permettant de détecter des activités enzymatiques différentes.

Le document WO-95/04157 décrit par exemple, des milieux de culture comportant différents substrats chromogènes d'osidases, tels que le 5-bromo-4-chloro-3-indoxyl galactoside, le 5-bromo-4-chloro-3-indoxyl glucuronide, le 5-bromo-4-chloro-3-indoxyl glucoside ou encore le 6-chloro-3-indoxyl galactoside. De tels milieux sont utilisés pour différencier notamment les bactéries de l'espèce *Escherichia coli* d'autres coliformes.

Ce document décrit également un milieu comportant des substrats chromogènes spécifiques de différentes enzymes, telles que des osidases et phosphatases. Ces substrats sont par exemple, le 5-bromo-4-chloro-3-indoxyl N-acétyl-glucosaminide et 5-bromo-6-chloro-3-indoxyl phosphate. Un tel milieu permet notamment de différencier *Candida albicans,* des autres levures du genre *Candida.*

Le document WO-00/53799 décrit un milieu chromogène de détection de bactéries *Staphylococcus aureus.* Ce milieu comporte deux substrats chromogènes en combinaison et notamment le 5-bromo-4-chloro-3-indoxyl glucoside et le 5-bromo-6-chloro-3-indoxyl phosphate. L'intérêt principal d'un tel milieu est d'éviter les faux positifs ou les faux négatifs, ainsi que de différencier les *S. aureus* d'autres espèces ou d'autres genres tels que les *Streptococcus*.

Toutefois, rien dans les documents WO-95/04157 et WO-00/53799 n'indique que les milieux décrits ont été élaborés afin d'améliorer la stabilité des substrats enzymatiques qu'ils contiennent.

Les Esters de Sorbitan et d'Acides Gras (ESAG) sont des tensioactifs connus, largement utilisés dans les préparations alimentaires et pharmaceutiques notamment. A titre illustratif, on peut citer l'article de *DICKINSON* et al. *: "J Colloid interface Sci 1999 Apr 15; 212 (2) : 466-473"* concernant la stabilisation d'émulsions contenant du Casénate de Sodium et un Monolauréate de Sorbitan polyoxyéthyléné comprenant 20 motifs oxyde d'éthylène (TWEEN 20). Les émulsions considérées sont des émulsions huile dans eau (30% volumique de n-tétradécane à pH 6,8). Ce domaine technique est relativement éloigné de celui de la détection de bactéries à activité enzymatique spécifique par réaction colorée sur milieu de culture sélectif.

Dans l'article *GRAM* et al *: "Clin Chem 1985 Oct; 31(10):1683-8",* il est question de mise en oeuvre de Monooléate de Sorbitan polyoxyéthyléné (TWEEN 80) dans un milieu de dosage de l'antitrombine-III, ce milieu comportant également du Polyéthylène Glycol, une solution de trombine et des substrats peptidiques chromogènes synthétiques. Les additifs mis en oeuvre sont présentés comme permettant l'augmentation de la concentration de thrombine active, de manière à garantir la bonne marche de l'analyse. Une fois de plus, force est de constater que ce genre de préoccupations techniques sont très distinctes de celles propres au milieu de détection de bactéries à activité enzymatique spécifique, à l'aide de milieux de culture sélectifs contenant des substrats chromogènes.

Dans un tel environnement technique, l'un des objectifs essentiels de la présente invention est de fournir un milieu de détection/identification de bactéries à activité enzymatique choisie parmi les activités estérases et/ou osidases et/ou peptidases et/ou sulfatases et/ou phosphatases, qui soit constitué de telle sorte que la sensibilité de l'analyse soit optimisée, c'est-à-dire que l'intensité de la coloration ou de la fluorescence révélant la présence de bactéries-cibles soit la plus forte possible.

Un autre objectif essentiel de la présente invention est de fournir un milieu de détection/identification de microorganismes, notamment de bactéries ou de levures, à activité enzymatique choisie parmi les activités estérases et/ou osidases et/ou peptidases et/ou sulfatases et/ou phosphatases, qui soit parfaitement translucide avant ensemencement par l'échantillon à analyser.

Un autre objectif essentiel de l'invention est de fournir un milieu de détection/identification de microorganismes, notamment de bactéries ou de levures à activité enzymatique choisie parmi les activités estérases et/ou osidases et/ou peptidases et/ou sulfatases et/ou phosphatases, qui comporte au moins un substrat d'enzyme choisi parmi les substrats d'estérases et/ou les substrats d'osidases et/ou les substrats de peptidases et/ou les substrats de sulfatases et/ou les substrats de phosphatases, chromogène ou fluorogène et qui soit stable à la conservation (intensité de la coloration ou de la fluorescence de révélation maintenue à un niveau maximum au moins pendant plusieurs semaines).

Un autre objectif essentiel de l'invention est de fournir un milieu de détection/identification de microorganismes, notamment de bactéries ou de levures à activité enzymatique choisie parmi les activités estérases et/ou osidases et/ou peptidases et/ou sulfatases et/ou phosphatases, qui ne se présente pas sous forme de poudre sèche à régénérer avec un liquide pour reconstituer un milieu liquide ou gélifié, mais qui existe directement sous des formes prêtes à l'emploi.

Un autre objectif essentiel de l'invention est de fournir un milieu de détection/identification de microorganismes, notamment de bactéries ou de levures à activité enzymatique choisie parmi les activités estérases et/ou osidases et/ou peptidases et/ou sulfatases et/ou phosphatases, qui soit économique, notamment du fait qu'il comprend des quantités réduites de substrat(s) d'enzyme, chromogène ou fluorogène, lequel a la particularité d'être coûteux.

Un autre objectif essentiel de l'invention est de fournir un procédé d'obtention du milieu de détection/identification sus visé qui soit simple à mettre en oeuvre et économique.

Un autre objectif essentiel de la présente invention est de fournir un procédé de détection/identification de souches à activité enzymatique choisie parmi les activités estérases, osidases et/ou peptidases et/ou sulfatases et/ou phosphatases, qui soit facile à mettre en oeuvre (tests de routine), qui soit économique (quantité de réactif, manipulation, main d'oeuvre, vitesse...) qui soit fiable, sensible, spécifique et reproductible.

Un autre objectif essentiel de la présente invention est de proposer l'utilisation d'un nouvel additif stabilisant dans des milieux de détection/identification de microorganismes, notamment les bactéries ou les levures, à activité enzymatique choisie parmi les activités estérases et/ou osidases et/ou peptidases et/ou sulfatases et/ou phosphatases, par révélation résultant d'une réaction d'hydrolyse libérant un colorant ou un produit fluorescent.

Ces objectifs, parmi d'autres, sont atteints par la présente invention qui concerne tout d'abord un milieu de détection/identification de microorganismes selon la revendication 1.

Un milieu de détection/identification de microorganismes et notamment de bactéries et/ou de levures à activité enzymatique choisie parmi les activités estérases et/ou osidases et/ou peptidases et/ou sulfatases et/ou phosphatases, selon l'invention, peut être obtenu à partir des milieux présentés dans les documents WO-95/04157 et WO-00/53799, décrits *supra.*

Le milieu selon l'invention présente l'avantage d'être un milieu prêt à l'emploi se présentant sous forme liquide ou semi-liquide (gel) sans que cela ne pose de problème quant à la stabilité. En effet, les substrats d'estérases et/ou d'osidases et/ou de peptidases et/ou de sulfatases et/ou de phosphatases, présents dans ce milieu et pourtant connus pour leur tendance à la dégradation relativement rapide, sont stabilisés grâce à la présence de l'additif stabilisant-émulsifiant ESAG, AG ou ESAG/AG.

Cet additif, éventuellement associé au solvant S, induit un autre avantage qui est de permettre une excellente dissolution des substrats d'estérases et/ou d'osidases et/ou de peptidases et/ou de sulfatases et/ou de phosphatases, traditionnellement récalcitrant à la mise en solution. Cela permet d'obtenir des milieux de détection/identification translucides avant l'ensemencement. La lecture des résultats colorés ou fluorescents s'en trouve ainsi facilitée.

Cet additif ESAG, AG ou ESAG/AG est peu onéreux et permet une simplification de la mise en oeuvre et une diminution de la quantité des substrats utilisée (stabilisation). Il en résulte un gain économique certain.

Enfin, les moyens selon l'invention sont à l'origine d'une meilleure activité biologique des substrats précités, ce qui se traduit par une coloration plus intense des colonies-cibles et par une meilleure spécificité.

Il est du mérite des inventeurs d'avoir sélectionné cette classe particulière d'agents stabilisant-émulsifiant, en particulier les ESAG, qui procurent de manière surprenante et inattendue des propriétés de stabilité, de translucidité, d'activité biologique, de sensibilité, de fiabilité et de spécificité, tout à fait bienvenues dans l'analyse microbiologique par voie biochimique selon l'invention.

Suivant une caractéristique remarquable de l'invention, le milieu de détection/identification qu'elle concerne, est obtenu par mélange d'au moins une solution mère de substrat dans le solvant S et de l'ESAG ou AG ou mélange ESAG/AG, ajouté à au moins une partie des constituants du milieu de culture.

Il est en effet apparu important conformément à l'invention de solubiliser le substrat d'estérases et/ou d'osidases et/ou de peptidases et/ou de sulfatases et/ou de phosphatases, dans le solvant S en présence d'un ou plusieurs ESAG, AG ou d'un mélange ESAG/AG répondant à la définition donnée ci-dessus. Ce mélange substrat ESAG, AG, ESAG/AG et solvant S est ensuite incorporé dans au moins une partie du milieu de culture, qui se présente, de préférence, sous forme gélifiée en surfusion. Il a pu être constaté que cette caractéristique opératoire permet une optimisation des résultats avantageux produits par les moyens selon l'invention.

De préférence, l'ESAG est sélectionné dans le groupe comprenant :
- Monolaurate de Sorbitan polyoxyéthylèné comprenant 20 motifs oxyde d'éthylène (O.E), -TWEEN® 20-;
- Monopalmitate de Sorbitan polyoxyéthylèné (20 O.E), -TWEEN® 40-;
- Monostéarate de Sorbitan polyoxyéthylèné (20 O.E), -TWEEN® 60-;
- Tristéarate de Sorbitan polyoxyéthylèné (20 O.E), -TWEEN® 65 -;
- Monooléate de Sorbitan polyoxyéthylèné (20 O.E), -TWEEN® 80-;
- Sesquioléate de Sorbitan polyoxyéthylèné (20 O.E), -TWEEN® 83-;
- Trioléate de Sorbitan polyoxyéthylèné (20 O.E), -TWEEN® 85-;
- et leurs mélanges ;
et l'AG est sélectionné dans le groupe comprenant les acides gras saturés ou insaturés en C4-C20, préférentiellement les acides gras saturés ou insaturés en C6-C11 et plus préférentiellement les acides gras en C8-C9, et leurs mélanges.

Les produits particulièrement sélectionnés, conformément à l'invention, sont des esters de Sorbitan largement utilisés dans l'industrie alimentaire et dans l'industrie cosmétique comme émulsifiants non ioniques, mais jusqu'alors ils n'avaient jamais été employés dans des milieux de détection/identification microbiologiques, à titre de stabilisants vis-à-vis de substrats d'estérases et/ou d'osidases et/ou de peptidases et/ou de sulfatases et/ou de phosphatases.

Les "Hydrophile-Lipophile-Balances" (HLB) des ESAG sus-mentionnés sont respectivement : 8,6 ; 6,7 ; 4,7 ; 2,1 ; 4,3 ; 3,7 ; 1,8.

Selon une variante intéressante de l'invention, le ou les agents stabilisant-émulsifiant décrits ci-dessus, en particulier le ou les ESAG, peuvent être associés à au moins un co-agent synergique, de préférence, au moins un tensioactif anionique, de préférence le 7-Ethyl-2-méthyl-4-undécylhydrogénosulfate ou ses sels et plus particulièrement ses sels de sodium (TERGITOL-4®).

L'utilisation d'un tel co-agent synergique facilite la mise en évidence de l'activité enzymatique dans les bactéries-cibles. Il s'agit d'un excellent complément du ou des agents stabilisant-émulsifiant et en particulier des ESAG. Il permet d'améliorer la sélectivité et la détection des activités estérases et/ou osidases et/ou peptidases et/ou sulfatases et/ou phosphatases des microorganismes-cibles, sans nuire à l'expression d'autres activités enzymatiques qui pourraient être éventuellement utilisées pour la mise en évidence des microorganismes-cibles considérés. Sans vouloir être lié par la théorie, il est supposé que le co-agent synergique TERGITOL-4® a une action qui favorise la pénétration des substrats d'estérases et/ou d'osidases et/ou de peptidases et/ou de sulfatases et/ou de phosphatases, chromogènes ou fluorogènes ou l'excrétion des enzymes au travers des membranes des cellules des microorganismes-cibles, optimisant ainsi l'accessibilité de ces substrats vis-à-vis des enzymes dont l'activité est recherchée. Cela permet de diminuer la quantité de substrats mis en oeuvre, d'où un avantage économique certain.

De manière plus préférée encore, l'association TWEEN® et TERGITOL-4® apparaît comme étant tout à fait performante dans le milieu de détection/identification selon l'invention.

S'agissant du substrat chromogène ou fluorogène, constitué d'une partie cible de l'enzyme et d'une partie chromophore ou fluorophore, il est avantageusement choisi parmi les substrats dont la partie cible est sélectionnée dans le groupe comprenant notamment :
- les glycosides, constitués par des unités mono-, di- et/ou polysaccharides, liés en α ou β à la fonction hydroxyle de la partie chromophore ou fluorophore;
- les acides α-aminés ou des peptides ;
- les acides organiques, tels que -O-CO(CH₂)ₙ-CH₃, avec n compris entre 0 et 20 ;
- un sulfate, un phosphate, un pyrosulfate, un pyrophosphate ou un phosphodiester ;
et dont la partie chromophore ou fluorophore est sélectionnée dans le groupe comprenant notamment :
- les Quinones/Anthraquinones et dérivés, notamment la Dihydroxyanthraquinone (Alizarine);
- l'amino- ou l'hydroxycoumarine et dérivés ;
- les fluorescéines et dérivés ;
- les Indoxyles et dérivés.

Comme exemples de substrats d'estérases, on peut citer les substrats esters chromogènes dérivés d'Indole et notamment le 5-Bromo-3-indolyl nonanoate, le 6-Chloro-3-indolyl nonaoate ou le 5-Bromo-3-indolyl décanoate.

A titre d'exemples de substrats d'estérases chromogènes dérivés de l'Anthraquinone, on peut citer le 2-Alizarine octanoate.

Comme exemples de substrats d'osidases, on peut citer les substrats d'osidases chromogènes dérivés d'Indole et notamment le 6-chloro-3-indolyl-β-galactoside, le 5-bromo-4-chloro-3-indolyl-β-N-acétylglucosaminide, le 5-bromo-6-chloro-α-glucoside.

A titre d'exemples de substrats d'osidases chromogènes dérivés de l'Anthraquinone, on peut citer le 2-Alizarine-β-glucuronide.

Comme substrats d'osidases fluorogènes, on peut citer les substrats dérivés d'hydroxycoumarine et notamment le 4-méthylumbelliférone-β-glucoside.

Concernant les substrats de peptidases chromogènes, on peut citer les substrats dérivés d'Indole.

Les substrats de peptidases fluorogènes sont notamment les substrats dérivés d'hydroxycoumarine, tels que l'analyl-amino-méthylcoumarine.

Comme substrats de sulfatases, on peut citer les substrats chromogènes, dérivés d'Indole, en particulier le 5-bromo-4-chloro-3-indolyl-sulfate. Les substrats de sulfatases fluorogènes sont par exemple le 4-méthylurnbelliférone-sulfate.

De façon équivalente, parmi les substrats de phosphatases, on peut citer des substrats chromogènes dérivés d'Indole tels que le 5-bromo-4-chloro-3-indolyl-phosphate. Les substrats de phosphatases fluorogènes sont par exemple le 4-méthylumbelliférone-phosphate.

Le solvant S est un auxiliaire de solubilisation du substrat d'enzymes d'intérêt en particulier chromogène. II complète également l'action de l'agent stabilisant émulsifiant ESAG, AG ou ESAG/AG. Il s'agit donc de préférence d'un constituant du milieu selon l'invention.

Selon une disposition avantageuse de cette dernière, le solvant S est sélectiormé dans le groupe comprenant :
- les alcools, de préférence le Méthanol, l'Ethanol, le Méthoxyéthanol,
- les amides, de préférence le DiMéthylFormamide (DMF),
- les solvants soufrés, de préférence le DiMéthylSulfOxyde (DMSO),
- les solvants aqueux, de préférence l'eau, l'eau tamponnée,
- et leurs mélanges.

En pratique, les solvants préférentiellement utilisés sont le méthanol, le DMF et le DMSO.

Sur le plan pondéral, il s'est avéré tout à fait avantageux, conformément à l'invention, que la proportion Ester de Sorbitan et d'Acide(s) Gras (ESAG) : Solvant (S) soit comprise entre 20 : 80 et 80 : 20, de préférence est comprise entre 30 : 70 et 70 : 30, et plus préférentiellement encore correspond à 40 : 60 ou à 60 : 40.

S'agissant du substrat chromogène ou fluorogène, les quantités mises en oeuvre sont telles que sa concentration dans le milieu est définie comme suit (en mg/l) :

| | |
|---|---|
| | 1 ≤ [substrat] ≤ 2000, |
| de préférence | 5 ≤ [substrat] ≤ 1500, |
| et plus préférentiellement encore | 25 ≤ [substrat] ≤ 1000. |

Concernant le substrat dans la solution d'ESAG et de solvants S, les quantités mises en oeuvre sont telles que sa concentration dans le milieu est définie comme suit (en g/l) :

| | |
|---|---|
| | 0,5 ≤ [substrat dans la solution mère] ≤ 2000, |
| de préférence | 1 ≤ [substrat dans la solution mère] ≤ 1000, |
| et plus préférentiellement encore | 2 ≤ [substrat dans la solution mère] ≤ 200. |

A propos du milieu de culture présent dans le milieu détection/identification, il peut être précisé qu'on le sélectionne dans le groupe comprenant:
- les milieux séléctifs de type : Mac Conkey, Columbia ANC, PALCAM, Sabouraud Gentamycine-Chloramphénicol, de préférence le milieu Mac Conkey,
- les milieux non sélectifs de type Columbia +/- sang, Trypcase Soja, Gélose nutritive, Sabouraud, de préférence le milieu Columbia.

En pratique, l'homme de l'art choisira le milieu de culture en fonction des bactéries-cibles, selon des critères parfaitement connus et à la portée de cet homme de l'art.

Sans que cela ne soit limitatif, il s'avère que le milieu selon l'invention est particulièrement adapté à la détection/identification de microorganismes d'intérêt médical ou industriel, et notamment ceux du genre *Salmonella, Pseudomonas, Listeria, Staphylococcus, Enterococcus* ou *Candida.*

Dans le cas de la détection de bactéries du genre *Salmonella*, on choisira par exemple, le milieu de Mac Conkey à titre de milieu de culture.

Par ailleurs, le milieu selon l'invention peut contenir d'éventuels autres additifs comme par exemple : un ou plusieurs autres substrats enzymatiques par exemple chromogènes ou fluorogènes, des peptones, un ou plusieurs facteurs de croissance, des hydrates de carbone, un ou plusieurs agents sélectifs, des tampons, et un ou plusieurs gélifiants.

Le milieu selon l'invention se présente sous forme de liquide ou de gel prêt à l'emploi, c'est-à-dire prêt à l'ensemencement en tube, flacon ou sur des boîtes de pétri.

Le milieu selon l'invention peut se conserver plusieurs semaines à 4°C dans ses conteneurs et sous forme liquide ou gel.

Selon un autre de ses aspects, la présente invention vise également un procédé d'obtention du milieu tel que défini ci-dessus caractérisé en ce qu'il consiste essentiellement :
- à préparer au moins une solution-mère du substrat d'estérases et/ou d'osidases et/ou de peptidases et/ou de sulfatases et/ou de phosphatases, chromogène ou fluorogène, dans le solvant S et d'au moins un ESAG, AG ou mélange ESAG/AG,
- à ajouter cette solution et d'éventuels autres additifs dans le milieu de culture, et
- à homogénéiser l'ensemble.

La préparation de la solution mère s'effectue de manière séparée en incorporant successivement le substrat, le solvant S et le ESAG, AG ou mélange ESAG/AG, éventuellement co-additivé. Les produits et les quantités utilisés sont tels que définis ci-dessus.

Après homogénéisation, la solution mère est ajoutée au milieu de culture gélifié mis en surfusion et préalablement régénéré dans l'eau. Il peut s'agir également d'un milieu liquide non gélifié, comme par exemple un bouillon nutritif.

Après mélange du milieu de culture et de la solution mère, on obtient le milieu de détection liquide ou gélifié prêt à être ensemencé.

Selon encore un autre de ses aspects, l'invention a également pour objet un procédé de détection/identification de souches à activité estérase et/ou osidase et/ou peptidase et/ou sulfatase et/ou phosphatase consistant à :
- ensemencer le milieu de détection/identification tel que défini ci-dessus en tant que produit *per se* ou en tant que produit obtenu par le procédé également décrit ci-dessus, avec l'échantillon contenant les bactéries cibles à analyser ;
- incuber le milieu ensemencé dans des conditions appropriées connues de l'homme de l'art ;
- et lire/interpréter les colorations ou fluorescences des colonies qui se sont développées après incubation, par exemple à l'étuve à 37°C ; ces colorations révélant l'hydrolyse du substrat d'estérases et/ou d'osidases et/ou de peptidases et/ou de sulfatases et/ou de phosphatases, chromogène ou fluorogène par les bactéries-cibles.

Enfin, l'invention concerne également l'utilisation d'Ester(s) de Sorbitan et d'Acide(s) Gras (ESAG), d'Acides Gras (AG) ou d'un mélange ESAG/AG, à titre d'agent stabilisant-émulsifiant de milieu (liquide ou gel) de détection/identification de microorganismes, notamment de bactéries ou de levures, à activité enzymatique choisie parmi les activités estérases et/ou osidases et/ou peptidases et/ou sulfatases et/ou phosphatases, ce milieu comprenant notamment un milieu réactionnel et au moins un substrat d'estérases et/ou d'osidases et/ou de peptidases et/ou de sulfatases et/ou de phosphatases, chromogène ou fluorogène, à l'exclusion des substrats comprenant un carboxylate de 4-[2-(4-octanoyloxy-3,5-diméthoxyphényl)-vinyl]-quinolinium-1-(propan-3-yle) et un anion, éventuellement en association avec au moins un solvant S du substrat d'estérases et/ou d'osidases et/ou de peptidases et/ou de sulfatases et/ou de phosphatases, chromogène ou fluorogène, présent dans le milieu.

Les exemples qui suivent permettront de mieux comprendre l'invention et d'appréhender tous ses avantages ainsi que ses diverses variantes de réalisation et de mise en oeuvre.

### EXEMPLES :

### Exemple 1 : essai de solubilité d'un substrat d'estérase à base d'Indoxyle, le 5-Bromo-3-indolyl nonanoate (substrat d'estérase chromogène), dans un milieu gélifié

Différentes solutions mères de substrat sont réalisées dans le Méthanol en présence de sels biliaires n°3 (bioMérieux). Ensuite, différents volumes de ces solutions sont ajoutés dans un milieu gélifié en surfusion : le milieu Mac Conkey (régénéré dans l'eau à une concentration de 50 g/l).

Le protocole et l'observation de l'aspect des milieux sont répertoriés dans le tableau 1 ci-après.

**TABLEAU 1**

| Milieu | 1 | 2 | 3 | 4 | 5 |
|---|---|---|---|---|---|
| Concentration de la solution mère en substrat | 100 g/l | 50 g/l | 25 g/l | 12,5 g/l | 6,25 g/l |
| Concentration finale en substrat dans le milieu | 500 mg/l | | | | |
| Concentration en sels biliaires dans la solution mère | 5 g/l | | | | 2,5 g/l |
| Concentration totale en sels biliaires dans le milieu | 7,5 g/l | | | | 4 g/l |
| Concentration en méthanol dans le milieu | 0,5 % | 1 % | 2 % | 4 % | 8 % |
| Observation du milieu coulé en boîtes | précipité sous forme de gros grains | précipité sous forme de grains plus petits que dans le milieu 1 | léger précipité : milieu laiteux | milieu laiteux | milieu laiteux |

Les milieux les plus optiquement homogènes sont les milieux 3, 4 et 5 : milieux où la concentration en méthanol est la plus élevée. Cependant, l'aspect de ces milieux est « laiteux » : il semblerait donc que le substrat d'estérase soit sous forme d'émulsion.

### Exemple 2 : amélioration de la solubilisation du substrat d'estérase par ajout de Tween ®20 (ESAG)

Deux solutions mères de substrat différentes sont réalisées dans le solvant S :Méthanol l'une en présence de sels biliaires n° 3, l'autre en présence de sels biliaires n° 3 et de Tween ®20 [monolaurate de sorbitan polyoxyéthylèné comprenant 20 motifs oxyde d'éthylène (O.E)]. Ensuite un volume correspondant à une concentration finale de 500 mg/l de substrat est ajouté dans un milieu de culture gélifié en surfusion : le milieu Mac Conkey. Le protocole et l'observation de l'aspect des milieux sont répertoriés dans le tableau 2 ci-après.

**TABLEAU 2**

| Milieu | 1 | 2 |
|---|---|---|
| Concentration de la solution mère en substrat | 100 g/l | 40 g/l |
| Concentration finale en substrat dans le milieu | 500 mg/l | 500 mg/l |
| Concentration en sels biliaires dans la solution mère | 5 g/l | 5 g/l |
| Concentration totale en sels biliaires dans le milieu | 6,5 g/l | 6,5 g/l |
| Concentration de Tween ®20 apporté à la solution mère de substrat | 0 % | 60 % |
| Concentration de Tween ®20 apporté au milieu | 0 % | 2,5 % |
| Observation du milieu coulé en boîtes | milieu laiteux | milieu translucide |

Le milieu 2 présente une meilleure homogénéité optique que le milieu 1. Soit le substrat d'estérase est complètement dissous, soit il est sous la forme d'une émulsion dont les particules micellaires ne sont plus visibles à l'oeil.

### Exemple 3 : test biologique des deux milieux décrits dans l'exemple précédent

Des micro-organismes issus de la collection de la Demanderesse ont été ensemencés sur chacun des deux milieux précédemment décrits par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 3 ci-dessous.

**TABLEAU 3**

| | temps | milieu 1 | | milieu 2 | |
|---|---|---|---|---|---|
| Souches | d'incubation | couleur | intensité | couleur | intensité |
| *Salmonella typhimurium* | 24 h | Gris | 1,5 | gris | 3 |
| 10 | 48 h | Gris | 1,5 | gris | 3,5 |
| *Salmonella paratyplai A* | 24 h | Gris | 1 | gris | 3 |
| 152 | 48 h | Gris | 1,5 | gris | 3,5 |
| *Proteus vulgaris* | 24 h | - | - | - | - |
| 15 | 48 h | - | - | - | - |
| *Serratia marcescens* | 24 h | Gris | 1,5 | gris | 3 |
| 13 | 48 h | Gris | 1 | gris | 4 |

| | | | | | |
|---|---|---|---|---|---|
| intensité de coloration : échelle arbitraire ; - : absence de coloration et d'intensité | | | | | |

Le milieu contenant le Tween®20 permet donc d'obtenir des colorations plus intenses pour les souches de micro-organismes présentant une activité estérase. Le substrat est donc plus utilisé soit parce qu'il est mieux dissous, soit parce qu'il est plus disponible. Vu la différence d'intensité entre les deux milieux, il semble possible, en présence de Tween®20, de diminuer la concentration en substrat utilisé et donc de réduire le coût engendré par l'utilisation de ce type de substrat.

### Exemple 4 : test de ce mode de dissolution pour un autre dérivé d'Indoxyle (substrat d'estérase chromogène) sur des espèces bactériennes à Gram positif et Gram négatif et sur des levures

Une solution mère à 40 g/l de 5-Bromo-4-chloro-3-indolyl octanoate (substrat d'estérase chromogène) a été réalisée dans un mélange Méthanol (40%) Tween®20 (60%). Ensuite un volume correspondant à une concentration finale de 500 mg/l de substrat a été ajouté dans un milieu gélifié en surfusion : un milieu type Columbia. Ce milieu a été coulé en boîtes de Pétri.

Des micro-organismes issus de la collection de la Demanderesse ont été ensemencés par isolement en trois cadrans à partir d'une suspension à 0,5 McFarland. Les boîtes ont ensuite été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 4 ci-dessous.

**TABLEAU 4**

| | temps | milieu 1 | |
|---|---|---|---|
| Souches | d'incubation | couleur | intensité |
| *Listeria monocytogenes* | 24 h | turquoise | 2,5 |
| 023 | 48 h | turquoise | 2,5 |
| *Listeria innocua* | 24 h | - | - |
| 029 | 48 h | turquoise | 0,5 |
| *Staphylococcus aureus* | 24 h | turquoise | 2,5 |
| 276 | 48 h | turquoise | 3 |
| *Candida albicans* | 24 h | turquoise | 0,5 |
| 033 | 48 h | turquoise | 3 |
| *Staphylococcus epidermidis* | 24 h | turquoise | 2 |
| 009 | 48 h | turquoise | 2 |
| *Salmonella spp.* | 24 h | turquoise | 2 |
| 017 | 48 h | turquoise | 3 |
| *Proteus vulgaris* | 24 h | - | - |
| 015 | 48 h | - | - |

| | | | |
|---|---|---|---|
| intensité de coloration : échelle arbitraire ; - : absence de coloration et d'intensité | | | |

En présence de Tween 20, il est donc possible de révéler l'expression d'une activité estérase chez tous les micro-organismes la possédant indépendamment du groupe auquel ils appartiennent. D'autre part, ce mode d'utilisation est indépendant du dérivé d'Indoxyle (substrat d'estérase chromogène) étudié.

### Exemple 5 : remplacement du Méthanol par d'autres solvants en présence de Tween 20 et en absence ou présence de sels biliaires

Le méthanol utilisé dans les exemples précédents a été remplacé par deux autres solvants le Diméthylsulfoxide (DMSO) et le Diméthylformamide (DMF). Les trois solvants ont été testés en présence de Tween 20 et en présence ou absence de sels biliaires à une concentration finale dans le milieu de 6,5 g/l. Le tableau 5 suivant montre la composition des six milieux testés.

**TABLEAU 5**

| Milieu | 1 | 2 | 3 | 4 | 5 | 6 |
|---|---|---|---|---|---|---|
| solvant S de la solution mère (SM) | Méthanol | DMSO | DMF | Méthanol | DMSO | DMF |
| % de solvant S dans la SM | 40 % | | | | | |
| % de Tween ®20 apporté à la SM de substrat | 60 % | | | | | |
| Concentration en sels biliaires dans le milieu | 6,5 g/l | | | 0 g/l | | |
| Concentration finale en substrat dans le milieu | 500 mg/l | | | | | |

L'activité du substrat a été vérifiée de la même façon que l'exemple précédent c'est à dire en présence de micro-organismes ensemencés sur un milieu gélosé Mac Conkey contenant le substrat d'estérase dissous dans un des solvants précédemment cités. Les résultats sont présentés dans le tableau 6 ci-dessous.

**TABLEAU 6**

| Milieu | | | 1 | | 2 | | 3 | | 4 | | 5 | | 6 | |
|---|---|---|---|---|---|---|---|---|---|---|---|---|---|---|
| Souches | | TI | C | I | C | I | C | I | C | I | C | I | C | I |
| *Salmonella* | | 24 | gris | 2 | gris | 2 | gris | 2 | gris | 2 | gris | 2 | gris | 2 |
| *Typhimurium* | 10 | 48 | gris | 2,5 | gris | 2,5 | gris | 2,5 | gris | 2,5 | gris | 2,5 | gris | 2,5 |
| *Salmonella* | | 24 | gris | 2 | gris | 2,5 | gris | 2 | gris | 2 | gris | 2,5 | gris | 2 |
| *arizonae* | 15 | 48 | gris | 2,5 | gris | 3 | gris | 2,5 | gris | 2,5 | gris | 3 | gris | 2,5 |
| *Pseudomonas* | | 24 | gris | 2 | gris | 2 | gris | 1 | gris | 2 | gris | 2 | gris | 1 |
| *aeruginosa* | 15 | 48 | gris | 2,5 | gris | 4 | gris | 3,5 | gris | 3 | gris | 4 | gris | 4 |
| *Proteus* | | 24 | - | - | - | - | - | - | - | - | - | - | - | - |
| *vulgaris* | 15 | 48 | - | - | - | - | - | - | - | - | - | - | - | - |
| *Serratia* | | 24 | gris | 2,5 | gris | 2 | gris | 2 | gris | 2,5 | gris | 2 | gris | 2 |
| *marcescens* | 38 | 48 | gris | 2,5 | gris | 2 | gris | 2 | gris | 2,5 | gris | 2 | gris | 2 |

| | | | | | | | | | | | | | | |
|---|---|---|---|---|---|---|---|---|---|---|---|---|---|---|
| I : intensité de coloration (échelle arbitraire) ; C : couleur ; TI : temps d'incubation en heures | | | | | | | | | | | | | | |
| - : incolore | | | | | | | | | | | | | | |

Il est donc possible d'utiliser des solvants différents pour dissoudre le substrat d'estérase lorsque le Tween 20 est présent. D'autre part, l'ajout de Sels biliaires n'apporte rien que ce soit en terme de solubilité ou en terme de résultats biologiques et ceci quel que soit le solvant testé. Conclusion, le mode de dissolution le plus simple est le mélange d'un solvant et de Tween ®20 sans apport supplémentaire de sels biliaires.

### Exemple 6 : test de différents ratios solvant S/Tween 20

Différentes solutions mères de substrat sont réalisées dans du DMSO en présence de Tween®20. Chaque solution mère correspond à un ratio DMSO/Tween® 20 différents. Ensuite un volume correspondant à une concentration finale de 500 mg/l de substrat est ajouté dans un milieu gélifié en surfusion : le milieu Mac Conkey. Le tableau 7suivant montre la composition des six milieux testés.

**TABLEAU 7**

| Milieu | 1 | 2 | 3 | 4 | 5 | 6 |
|---|---|---|---|---|---|---|
| % de DMSO dans la solution mère | 10 % | 20 % | 30 % | 40 % | 50 % | 60 % |
| % Tween ®20 apporté à la solution mère de substrat | 90 % | 80 % | 70 % | 60 % | 50 % | 40 % |

Des micro-organismes issus de la collection de la Demanderesse ont été ensemencés sur chacun des six milieux précédemment décrits par isolement en trois cadrans à partir d'une suspension à 0,5 McFarland. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 8 ci-après.

**TABLEAU 8**

| Milieu | | | 1 | | 2 | | 3 | | 4 | | 5 | | 6 | |
|---|---|---|---|---|---|---|---|---|---|---|---|---|---|---|
| Souches | | TI | C | I | C | I | C | I | C | I | C | I | C | I |
| *Salmonella* | | 24 | gris | 2,5 | gris | 2,5 | gris | 2 | gris | 3 | gris | 3 | gris | 3 |
| *typhimurium* | 10 | 48 | gris | 3 | gris | 3 | gris | 2,5 | gris | 3,5 | gris | 3 | gris | 3,5 |
| *Salmonella* | | 24 | gris | 2,5 | gris | 2,5 | gris | 3 | gris | 3,5 | gris | 3 | gris | 3,5 |
| *arizonae* | 15 | 48 | gris | 2,5 | gris | 2,5 | gris | 3 | gris | 3,5 | gris | 3 | gris | 3,5 |
| *Serratia* | | 24 | gris | 3 | gris | 3 | gris | 3 | gris | 4 | gris | 3,5 | gris | 3,5 |
| *marcescens* | 045 | 48 | gris | 4 | gris | 4 | gris | 4 | gris | 4 | gris | 4 | gris | 4 |
| *Hafnia* | | 24 - | - | - | - | - | - | - | - | - | - | - | - | - |
| *alvei* | 025 | 48 | - | - | - | - | - | - | - | - | - | - | - | - |
| *Pseudomonas* | | 24 | gris | 2 | gris | 2 | gris | 3 | gris | 3 | gris | 3 | gris | 3 |
| *aeruginosa* | 165 | 48 | gris | 2 | gris | 2 | gris | 2 | gris | 3 | gris | 3 | gris | 3 |
| *Salmonella* | | 24 | gris | 1,5 | gris | 1,5 | gris | 2 | gris | 3 | gris | 2,5 | gris | 3 |
| *paratyphi A* | 006 | 48 | gris | 2 | gris | 2 | gris | 2 | gris | 3 | gris | 3 | gris | 3 |
| *Salmonella* | | 24 | gris | 2 | gris | 2 | gris | 2 | gris | 3 | gris | 2 | gris | 3 |
| *typhi* | 118 | 48 | gris | 2 | gris | 2 | gris | 2 | gris | 3 3 | gris | 2 | gris | 3 |

| | | | | | | | | | | | | | | |
|---|---|---|---|---|---|---|---|---|---|---|---|---|---|---|
| I : intensité de coloration (échelle arbitraire) ; C : couleur ; TI : temps d'incubation en heures | | | | | | | | | | | | | | |
| - : incolore | | | | | | | | | | | | | | |

Les six ratios testés permettent d'obtenir une bonne solubilité des substrats et la révélation de l'hydrolyse du substrat avec des intensités de coloration très convenables. Des intensités de coloration maximales sont obtenues entre les ratios 40 % DMSO /60 % Tween ®20 et 60 % DMSO / 40 % Tween ®20.

### Exemple 7 : test du procédé de solubilisation sur un substrat d'estérase ayant une partie marqueur autre qu'un Indoxyle

Le substrat testé est le 1,2-Dihydroxyanthraquinone octanoate (2-Alizarine octanoate). Différentes solutions mères de substrat sont réalisées avec comme solvant le DMF ou le DMSO ou le méthanol ou le Méthoxyéthanol et dans tous les cas en présence de Tween® 20. Le tableau 9 suivant montre l'aspect des solutions mères en fonction de leur composition.

**TABLEAU 9**

| | | | | |
|---|---|---|---|---|
| solvant de la solution mère | Méthanol | DMSO | DMF | Méthoxyéthanol |
| % de solvant dans la solution mère | 40 % | | | |
| % de Tween ®20 apporté à la solution mère de substrat | 60 % | | | |
| aspect de la solution mère | précipité | précipité | dissolution totale | dissolution totale |

Ensuite un volume correspondant à une concentration finale de 100 mg/l de substrat est ajouté dans un milieu gélifié en surfusion : le milieu Mac Conkey. Du Citrate de fer à une concentration finale de 50 mg/l a été aussi ajouté dans le milieu ceci afin de permettre la révélation de l'activité estérase en présence d'un substrat à base d'Alizarine. Seule une des solutions mères présentant une dissolution totale du substrat a été testée (solution mère dans le DMF). Des micro-organismes issus de la collection de la Demanderesse ont été ensemencés sur chacun des deux milieux précédemment décrits par isolement en trois cadrans à partir d'une suspension à 0,5 McFarland. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 10.

**TABLEAU 10**

| | | Temps | milieu avec DMF | |
|---|---|---|---|---|
| Souches | | d'incubation | couleur | intensité |
| *Salmonella typhimurium* | | 24 h | mauve | 1 |
| | 10 | 48 h | mauve | 2 |
| *Salmonella enteritidis* | | 24 h | mauve | 1 |
| | 036 | 48 h | mauve | 3 |
| *Salmonella arizonae* | | 24 h | mauve | 1 |
| | 018 | 48 h | mauve | 2 |
| *Escherichia coli* | | 24 h | - | - |
| | 002 | 48 h | - | - |
| *Serratia marcescens* | | 24 h | mauve | 3,5 |
| | 045 | 48 h | mauve | 3,5 |
| *Stapliylococcus aureus* | | 24 h | mauve | 3 |
| | 033 | 48 h | mauve | 4 |
| *Pseudomonas aeruginosa* | | 24 h | mauve | 2 |
| | 165 | 48 h | mauve | 2 |
| *Salmonella gallinarum* | | 24 h | mauve | 0,5 |
| | 500 | 48 h | mauve | 3 |

| | | | | |
|---|---|---|---|---|
| intensité de coloration : échelle arbitraire ; - : absence de coloration et d'intensité. | | | | |

Ce mode de dissolution n'est donc pas spécifique des esters d'indoxyle mais peut être utilisé notamment avec d'autres familles de substrats enzymatiques.

### Exemple 8: test de différents ESAG dérivés de Tween® 20 pour la solubilisation d'un substrat d'estérase, le 5-Bromo-3-indolyl nonanoate

Différentes solutions mères de substrat sont réalisées dans du DMSO en présence de Tween® 20 ou Tween® 80 (Polyoxyéthylène sorbitan monooléate) ou Tween® 60 (Polyoxyethyléne sorbitan monostéarate) ou Tween® 65 (Polyoxyéthylène sorbitan tristéarate). Ensuite un volume correspondant à une concentration finale de 500 mg/l de substrat est ajouté dans un milieu gélifié en surfusion : le milieu Mac Conkey. Le tableau 11 montre la composition des milieux testés.

**TABLEAU 11**

| Milieu | 1 | 2 | 3 | 4 |
|---|---|---|---|---|
| % de DMSO dans la solution mère | 40 % | 40 % | 40 % | 40 % |
| % de Tween 20 apporté à la solution mère de substrat | 60 % | - | - | - |
| % de Tween 60 apporté à la solution mère de substrat | - | 60 % | - | - |
| % de Tween 65 apporté à la solution mère de substrat | - | - | 60 % | - |
| % de Tween 80 apporté à la solution mère de substrat | - | - | - | 60 % |

Des micro-organismes issus de la collection de la Demanderesse ont été ensemencés sur chacun des six milieux précédemment décrits par isolement en trois cadrans à partir d'une suspension à 0,5 McFarland. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 12.

**TABLEAU 12**

| Milieu | | 1 | | 2 | | 3 | | 4 | |
|---|---|---|---|---|---|---|---|---|---|
| Souches | TI | C | I | C | I | C | I | C | I |
| *Salmonella typhimurium* | 24 h | gris | 2,5 | gris | 2 | - | - | gris | 2 |
| 10 | 48 h | gris | 3 | gris | 2 | - | - | gris | 2 |
| *Salmonella arizonae* | 24 h | gris | 2,5 | gris | 1 | - | - | gris | 1 |
| 15 | 48 h | gris | 3 | gris | 2 | - | - | gris | 3 |
| *Salmonella paratyphi A* | 24 h | gris | 1,5 | gris | 1 | - | - | gris | 1 |
| 006 | 48 h | gris | 3,5 | gris | 2 | - | - | gris | 3 |
| *Salmonella typhi* | 24 h | gris | 2 | gris | 1 | - | - | gris | 1 |
| 118 | 48 h | gris | 2,5 | gris | 2 | - | - | gris | 2 |
| *Pseudomonas aeruginosa* | 24 h | gris | 3 | gris | 0,5 | - | - | gris | 4 |
| 165 | 48 h | gris | 3 | gris | 2 | - | - | gris | 4 |
| *Hafnia alvei* | 24 h | - | - | - | - | - | - | - | - |
| 025 | 48 h | - | - | - | - | - | - | - | - |

| | | | | | | | | | |
|---|---|---|---|---|---|---|---|---|---|
| I : intensité de coloration (échelle arbitraire) ; C : couleur ; TI : temps d'incubation - : incolore | | | | | | | | | |

Remarque : Différents ratio Tween®/solvant ont été réalisés pour chaque Tween® testés. Cependant, seul un ratio est répertorié dans cet exemple car les résultats étaient très similaires quel que soit le ratio étudié.

Le Tween® 20, le Tween® 80 et le Tween® 60 permettent d'obtenir une bonne solubilité des substrats et la révélation de l'hydrolyse du substrat avec des intensités de coloration très convenables.

### Exemple 9 : stabilité comparée de milieux gélifiés contenant un substrat d'estérase, le 5-Bromo-3-indolyl nonanoate, solubilisé ou non en présence de Tween® 20

Deux solutions mères de substrat ont été réalisées : l'une à 40 g/l dans le méthanol en présence de 6,5 g/l de sels biliaires n° 3, l'autre à 40 g/l dans un mélange DMSO (40%) Tween® 20 (60%). Ensuite un volume correspondant à une concentration finale de 500 mg/l de substrat a été ajouté dans un milieu gélifié en surfusion, le milieu Mac Conkey. Ces deux milieux ont été coulés en boîtes de Pétri d'un diamêtre de 90 mm et conservés à +4°C pendant 8 semaines. Après une semaine, deux semaines, quatre semaines et huit semaines, deux milieux comme ceux décrits ci-dessus ont été réalisés extemporanément.

Des micro-organismes issus de la collection de la Demanderesse ont été ensemencés par isolement en trois cadrans à partir d'une suspension à 0,5 McFarland sur les deux milieux préparés extemporanément et les deux milieux conservés à +4°C. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans les tableaux ci-après.

**TABLEAU 13**

| Milieu | | Frais | | conservé **1** semaine | | frais | | conservé **1** semaine | |
|---|---|---|---|---|---|---|---|---|---|
| | | méthanol sels biliaires | | méthanol sels biliaires | | DMSO Tween 20 | | DMSO Tween 20 | |
| Souches | TI | C | I | C | I | C | I | C | I |
| *Salmonella typhimurium* | 24 h | gris | 0,5 | gris | 0,5 | gris | 3 | gris | 3 |
| 10 | 48 h | gris | 1,5 | gris | 1,5 | gris | 4 | gris | 4 |
| *Salmonella arizonae* | 24 h | gris | 1 | gris | 1 | gris | 3 | gris | 3 |
| 15 | 48 h | gris | 1,5 | gris | 1,5 | gris | 4 | gris | 4 |
| *Salmonella paratyphi A* | 24 h | - | - | - | - | gris | 2 | gris | 2 |
| 006 | 48 h | gris | 0,5 | gris | 0,5 | gr | is 4 | gris | 4 |
| *Salmonella typhi* | 24 h | gris | 0,5 | gris | 0,5 | gris | 3 | gris | 3 |
| 118 | 48 h | gris | 1,5 | gris | 1,5 | gris | 4 | gris | 4 |
| *Pseudomonas aeruginosa* | 24 h | gris | 1 | gris | 1 | gris | 2 | gris | 2 |
| 165 | 48 h | gris | 1 | gris | 1 | gris | 3 | gris | 3 |
| *Hafnia alvei* | 24 h | - | - | - | - | - | - | - | - |
| 025 | 48 h | - | - | - | - | - | - | - | - |

**TABLEAU 14**

| Milieu | | Frais | | conservé **2** semaines | | frais | | conservé **2** semaines | |
|---|---|---|---|---|---|---|---|---|---|
| | | méthanol sels biliaires | | Méthanol sels biliaires | | DMSO Tween 20 | | DMSO Tween 20 | |
| Souches | TI | C | I | C | I | C | I | C | I |
| *Salmonella typhimurium* | 24 h | gris | 0,5 | gris | 0,5 | gris | 3 | Gris | 3 |
| 10 | 48 h | gris | 1,5 | gris | 1,5 | gris | 4 | Gris | 4 |
| *Salmonella arizonae* | 24 h | gris | 1 | gris | **0,1** | gris | 3 | Gris | 3 |
| 15 | 48 h | gris | 1,5 | gris | **0,5** | gris | 4 | Gris | 4 |
| *Salmonella paratyphi A* | 24 h | - | - | - | - | gris | 2 | Gris | 2 |
| 006 | 48 h | gris | 0,5 | gris | **0,1** | girs | 4 | Gris | 4 |
| *Salmonella typhi* | 24 h | gris | 0,5 | - | - | gris | 3 | gris | 3 |
| 118 | 48 h | gris | 1,5 | gris | **0,5** | gris | 4 | gris | 4 |
| *Pseudomonas aeruginosa* | 24 h | gris | **1** | gris | 1 | gris | 2 | gris | 2 |
| 165 | 48 h | gris | 1 | gris | **1** | gris | 3 | gris | 3 |
| *Hafnia alvei Hafnia alvei* | 24 h | - | - | - | - | - | - | - | - |
| 025 | 48 h | - | - | - | - | - | - | - | - |

**TABLEAU 15**

| Milieu | | Frais | | conservé **4** semaines | | frais | | conservé **4** semaines | |
|---|---|---|---|---|---|---|---|---|---|
| | | méthanol sels biliaires | | Méthanol sels biliaires | | DMSO Tween 20 | | DMSO Tween 20 | |
| Souches | TI | C | I | C | I | C | I | C | I |
| *Salmonella typhimurium* | 24 h | gris | 0,5 | - | - | gris | 3 | gris | 3 |
| 10 | 48 h | gris | 1,5 | gris | **0,5** | gris | 4 | gris | 4 |
| *Salmonella arizonae* | 24 h | gris | 1 | gris | **0,1** | gris | 3 | gris | 3 |
| 15 | 48 h | gris | 1,5 | gris | **0,5** | gris | 4 | gris | 4 |
| *Salmonella paratyphi A* | 24 h | - | - | - | - | gris | 2 | gris | 2 |
| 006 | 48 h | gris | 0,5 | gris | **0,1** | gris | 4 | gris | 4 |
| *Salmonella typhi* | 24 h | gris | 0,5 | - | - | gris | 3 | gris | 3 |
| 118 | 48 h | gris | 1,5 | gris | **0,5** | gris | 4 | gris | 4 |
| *Pseudomonas aeruginosa* | 24 h | gris | 1 | gris | **0,5** | gris | 2 | gris | 2 |
| 165 | 48 h | gris | 1 | gris | **1** | gris | 3 | gris | 3 |
| *Hafnia alvei* | 24 h | - | - | - | - | - | - | - | - |
| 025 | 48 h | - | - | - | - | - | - | - | - |

**TABLEAU 16**

| Milieu | | Frais | | conservé **8** semaines | | frais | | conservé **8** semaines | |
|---|---|---|---|---|---|---|---|---|---|
| | | méthanol sels biliaires | | Méthanol sels biliaires | | DMSO Tween 20 | | DMSO Tween 20 | |
| Souches | TI | C | I | C | I | C | I | C | I |
| *Salmonella typhimurium* | 24 h | gris | 0,5 | - | - | gris | 3 | gris | **2,5** |
| 10 | 48 h | gris | 1,5 | gris | **0,5** | gris | 4 | gris | **3,5** |
| *Salmonella arizonae* | 24 h | gris | 1 | - | - | gris | 3 | gris | **3** |
| 15 | 48h | gris | 1,5 | gris | **0,5** | gris | 4 | gris | **3** |
| *Salmonella paratyphi A* | 24 h | - | - | - | - | gris | 2 | gris | **1,5** |
| 006 | 48 h | gris | 0,5 | - | - | gris | 4 | gris | **3** |
| *Salmonella typhi* | 24 h | gris | 0,5 | - | - | gris | 3 | gris | **2** |
| 118 | 48 h | gris | 1,5 | gris | **0,5** | gris | 4 | gris | **3** |
| *Pseudomonas aeruginosa* | 24 h | gris | 1 | gris | **0,5** | gris | 2 | gris | 2 |
| 165 | 48 h | gris | 1 | gris | **1** | gris | 3 | gris | 3 |
| *Hafnia alvei* | 24 h | - | - | - | - | - | - | - | - |
| 025 | 48 h | - | - | - | - | - | - | - | - |

| | | | | | | | | | |
|---|---|---|---|---|---|---|---|---|---|
| I : intensité de coloration (échelle arbitraire) ; C : couleur ; TI : temps d'incubation - : incolore | | | | | | | | | |

Le milieu contenant le substrat dissous dans un mélange Méthanol sels biliaires présente une baisse très importante d'expression de l'activité estérase dès 2 semaines de conservation. Cette baisse est amplifiée dans le temps. Le milieu contenant le substrat dissous dans le mélange DMSO et Tween 20 présente une expression de l'activité estérase constante dans le temps jusqu'au moins six semaines. A huit semaines, il y a une légère baisse de l'expression de l'activité estérase mais qui n'est pas rédhibitoire. Le Tween 20 permet donc de stabiliser le substrat d'estérase et facilite la conservation et l'utilisation de milieux de culture contenant ce type de substrats.

Note : Dans les exemples ci-dessus, les numéros annexés aux souches correspondent au numéro de chaque souche référencée dans la collection de la Demanderesse. L'intensité de coloration correspond à une échelle arbitraire dont la signification est la suivante :

| | |
|---|---|
| 0 | pas d'activité |
| 0,1 | trace de coloration |
| 0,5 | coloration très pâle |
| 1 | coloration nette de faible intensité |
| 2 | coloration franche d'intensité moyenne |
| 3 | coloration intense |
| 4 | coloration très intense |

### Exemple 10 : stabilité comparée de milieux gélifiés contenant un substrat d'osidase, le 6-Chloro-3-indoly-β-n-acétylglucosaminide, solubilisé ou non en présence de Tween® 20

Deux solutions mères de substrat ont été réalisées : l'une à 50 g/l dans le DMF, l'autre à 50 g/l dans un mélange DMF (40%) Tween® 20 (60%). Ensuite un volume correspondant à une concentration finale de 175 mg/l de substrat a été ajouté dans un milieu gélifié en surfusion, le milieu Columbia sans sang. Ces deux milieux ont été coulés en boîtes de Pétri d'un diamètre de 90 mm et conservés à +4°C pendant 8 semaines. Après trois semaines, six semaines, neuf semaines et douze semaines, deux milieux comme ceux décrits ci-dessus ont été réalisés extemporanément.

Des micro-organismes issus de la collection de la Demanderesse ont été ensemencés par isolement en trois cadrans à partir d'une suspension à 0,5 McFarland sur les deux milieux préparés extemporanément et les deux milieux conservés à +4°C. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans les tableaux ci-dessous.

**TABLEAU 17**

| Milieu | | frais | | conservé **3** semaine | | frais | | conservé **3** semaine | |
|---|---|---|---|---|---|---|---|---|---|
| | | DMF | | DMF | | DMF Tween 20 | | DMF Tween 20 | |
| Souches | TI | C | I | C | I | C | I | C | I |
| *Listeria innocua* | 24 h | M | 2 | M | **1,5** | M | 2 | M | 2 |
| 36 | 48 h | M | 3 | M | 3 | M | 3 | M | 3 |
| *Listeria ivanovii* | 24 h | M | 1,5 | M | **1** | M | 1,5 | M | 1,5 |
| 18 | 48 h | M | 2,5 | M | 2,5 | M | 2,5 | M | 2,5 |
| *Listeria monocytogenes* | 24 h | M | 2 | M | **1,5** | M | 2 | M | 2 |
| 23 | 48 h | M | 3 | M | 3 | M | 3 | M | 3 |
| *Staphylococcus aureus* | 24 h | - | - | - | - | - | - | - | - |
| 20 | 48 h | - | - | - | - | - | - | - | - |
| *Pseudomonas aeruginosa* | 24 h | - | - | - | - | - | - | - | - |
| 165 | 48 h | - | - | - | - | - | - | - | - |

**TABLEAU 18**

| Milieu | | frais | | conservé **6** semaines | | frais | | conservé **6** semaines | |
|---|---|---|---|---|---|---|---|---|---|
| | | DMF | | DMF | | DMF Tween 20 | | DMF Tween 20 | |
| Souches | TI | C | I | C | I | C | I | C | I |
| *Listeria innocua* | 24 h | M | 2 | M | **1** | M | 2 | M | 2 |
| 36 | 48 h | M | 3 | M | **2** | M | 3 | M | 3 |
| *Listeria ivanovii* | 24 h | M | 1,5 | M | **0,5** | M | 1,5 | M | 1,5 |
| 18 | 48 h | M | 2,5 | M | **2** | M | 2,5 | M | 2,5 |
| *Listeria monocytogenes* | 24 h | M | 2 | M | **1** | M | 2 | M | 2 |
| 23 | 48 h | M | 3 | M | **2** | M | 3 | M | 3 |
| *Staphylococcus aureus* | 24 h | - | - | - | - | - | - | - | - |
| 20 | 48 h | - | - | - | - | - | - | - | - |
| *Pseudomonas aeruginosa* | 24 h | - | - | - | - | - | - | - | - |
| 165 | 48h | - | - | - | - | - | - | - | - |

**TABLEAU 19**

| Milieu | | frais | | conservé **9** semaines | | frais | | conservé **9** semaines | |
|---|---|---|---|---|---|---|---|---|---|
| | | DMF | | DMF | | DMF Tween 20 | | DMF Tween 20 | |
| Souches | TI | C | I | C | I | C | I | C | I |
| *Listeria innocua* | 24 h | M | 2 | M | **0,5** | M | 2 | M | **1,5** |
| 36 | 48 h | M | 3 | M | **1** | M | 3 | M | **2,5** |
| *Listeria ivanovii* | 24 h | M | 1,5 | M | - | M | 1,5 | M | **1** |
| 18 | 48 h | M | 2,5 | M | **0,5** | M | 2,5 | M | **2,5** |
| *Listeria monocytogenes* | 24 h | M | 2 | M | **0,5** | M | 2 | M | **1** |
| 23 | 48 h | M | 3 | M | **0,5** | M | 3 | M | **2** |
| *Staphylococcus aureus* | 24 h | - | - | - | - | - | - | - | - |
| 20 | 48 h | - | - | - | - | - | - | - | - |
| *Pseudomonas aeruginosa* | 24 h | - | - | - | - | - | - | - | - |
| 165 | 48 h | - | - | - | - | - | - | - | - |

**TABLEAU 20**

| Milieu | | frais | | conservé **12** semaines | | frais | | conservé **12** semaines | |
|---|---|---|---|---|---|---|---|---|---|
| | | DMF | | DMF | | DMF Tween 20 | | DMF Tween 20 | |
| Souches | TI | C | I | C | I | C | I | C | I |
| *Listeria innocua* | 24 h | M | 2 | M | - | M | 2 | M | **1** |
| 36 | 48h | M | 3 | M | **0,5** | M | 3 | M | **2,5** |
| *Listeria ivanovii* | 24 h | M | 1,5 | M | - | M | 1,5 | M | **1** |
| 18 | 48 h | M | 2,5 | M | - | M | 2,5 | M | 2 |
| *Listeria monocytogenes* | 24 h | M | 2 | M | - | M | 2 | M | 1 |
| 23 | 48 h | M | 3 | M | - | M | 3 | M | **2** |
| *Staphylococcus aureus* | 24 h | - | - | - | - | - | - | - | - |
| 20 | 48 h | - | - | - | - | - | - | - | - |
| *Pseudomonas aeruginosa* | 24 h | - | - | - | - | - | - | - | - |
| 165 | 48 h | - | - | - | - | - | - | - | - |

| | | | | | | | | | |
|---|---|---|---|---|---|---|---|---|---|
| I : intensité de coloration (échelle arbitraire) ; C : couleur ; TI : temps d'incubation | | | | | | | | | |
| M : magenta | | | | | | | | | |
| - : incolore | | | | | | | | | |

Le milieu contenant le substrat dissous dans le DMF seul présente une baisse très importante d'expression de l'activité β-N-acétylglucosaminidase dès trois semaines de conservation. Cette baisse est amplifiée dans le temps. Il n'est plus possible de détecter une activité à douze semaines. Le milieu contenant le substrat dissous dans le mélange DMF et Tween 20 présente une expression de l'activité β-N-acétylglucosaminidase constante dans le temps jusqu'à au moins six semaines. A neuf et douze semaines, il y a une légère baisse de l'expression de l'activité β-N-acétylglucosaminidase mais qui n'est pas rédhibitoire. Le Tween 20 permet donc de stabiliser le substrat de β-N-acétylglucosaminidase et facilite la conservation et l'utilisation de milieux de culture contenant ce type de substrats.

Dans les exemples ci-dessus, les numéros annexés aux souches correspondent au numéro de chaque souche référencée dans la collection de la Demanderesse. L'intensité de coloration correspond à une échelle arbitraire dont la signification est la suivante :

| | |
|---|---|
| 0 | pas d'activité |
| 0,1 | trace de coloration |
| 0,5 | coloration très pâle |
| 1 | coloration nette de faible intensité |
| 2 | coloration franche d'intensité moyenne |
| 3 | coloration intense |
| 4 | coloration très intense |

## Revendications

1. Milieu de détection/identification de microorganismes et notamment de bactéries et/ou de levures à activité enzymatique, choisie parmi les activités estérases et/ou osidases et/ou peptidases et/ou sulfatases et/ou phosphatases, du type de ceux comprenant notamment un milieu réactionnel et au moins un substrat d'estérases, d'osidases et/ou de peptidases et/ou de sulfatases et/ou de phosphatases, chromogène ou fluorogène, à l'exclusion des substrats comprenant un cation de 4-[2-(4-octanoyloxy-3,5-diméthoxyphényl)-vinyl]-quinolinium-1-(propan-3-yle-acide carboxylique) et un anion,
cette détection/identification reposant essentiellement sur la mise en évidence de l'activité estérase et/ou osidase et/ou peptidase et/ou sulfatase et/ou phosphatase,
ledit milieu :
* est sous forme liquide ou gel prêt à l'emploi et stable à la conservation, c'est-à-dire, l'intensité de la coloration ou de la fluorescence est maintenue à un niveau maximum au moins pendant plusieurs semaines;
* comporte :
- au moins un Ester de Sorbitan et d'Acide(s) Gras (ESAG), ou au moins un Acide Gras (AG) ou un mélange ESAG/AG, à titre d'agent stabilisant-émulsifiant,
- la concentration en [ESAG] dans le milieu est définie comme suit (en % en poids): 0,5 ≤ [ESAG] ≤ 5, de préférence 1,5 ≤ [ESAG] ≤ 3,5;
- et éventuellement au moins un Solvant (S).

2. Milieu selon la revendication 1, **caractérisé en ce qu'**il est obtenu par mélange d'au moins une solution-mère de substrat dans le solvant S et de l'ESAG, AG ou mélange ESAG/AG, avec au moins une partie des constituants du milieu de culture.

3. Milieu selon la revendication 1 ou 2, **caractérisé en ce que** l'ESAG est sélectionné dans le groupe comprenant:
* Monolaurate de Sorbitan polyoxyéthylèné comprenant 20 motifs oxyde d'éthylène (O.E), -TWEEN® 20-;
* Monopalmitate de Sorbitan polyoxyéthylèné (20 O.E), -TWEEN® 40-;
* Monostéarate de Sorbitan polyoxyéthylèné (20 O.E), -TWBEN® 60-;
* Tristéarate de Sorbitan polyoxyéthyièné (20 O.E), -TWEEN® 65-:
* Monooléate de Sorbitan polyoxyéthylèné (20 O.E), -TWEEN® 80-;
* Sesquioléate de Sorbitan polyoxyéthylèné (20 O.E), -TWEEN® 83-;
* Trioléate de Sorbitan polyoxyéthylèné (20 O.E), -TWEEN® 85-;
* et leurs mélanges.

4. Milieu selon la revendication 1 ou 2, **caractérisé en ce que** l'AG est sélectionné dans le groupe comprenant les acides gras saturés ou insaturés en C4-C20, préférentiellement les acides gras saturés ou insaturés en C6-C11 et plus préférentiellement les acides gras en C8-C9; et leurs mélanges.

5. Milieu selon l'une quelconque des revendications 1 à 4, **caractérisé en ce qu'**il comprend également au moins un tensioactif anionique, de préférence le 7-Ethyl-2-méthyl-4-undécylhydrogénosulfate ou ses sels et plus particulièrement ses sels de sodium (TERG1TOL-4 ®).

6. Milieu selon l'une quelconque des revendications 1 à 5, **caractérisé en ce que** le substrat chromogène ou fluorogène, est constitué d'une partie cible de l'enzyme et d'une partie chromophore ou fluorophore, la partie cible étant sélectionnée dans le groupe comprenant notamment:
- les glycosides, constitués par des unités mono-, di- et/ou polysaccharides, liés en α ou β à la fonction hydroxyle de la partie fluorophore ou chromophore;
- les acides α-aminés ou des peptides ;
- les acides organiques, tels que -O-CO(CH₂)ₙ-CH₃, avec n compris entre 0 et 20 ;
- un sulfate, un phosphate, un pyrosulfate, un pyrophosphate ou un phosphodiester ;
la partie chromophore ou fluorophore étant sélectionnée dans le groupe comprenant notamment :
- les Quinones/Anthraquinones et dérivés, notamment la Dihydroxyanthraquinone (Alizarine);
- les amino- ou hydroxy- coumarines et dérivés ;
- les fluorescéines et dérivés ;
- les Indoxyles et dérivés.

7. Milieu selon l'une quelconque des revendications 1 à 6, **caractérisé en ce que** le solvant S est sélectionné dans le groupe comprenant :
- les alcools, de préférence le Méthanol, l'Ethanol, le Méthoxyéthanol ;
- les amides, de préférence le DiMéthylFormamide (DMF) ;
- les solvants soufrés, de préférence le DiMéthylSulfOxyde (DMSO) ;
- les solvants aqueux, de préférence l'eau, l'eau tamponnée ;
- et leurs mélanges.

8. Milieu selon l'une quelconque des revendications 1 à 7, **caractérisé en ce que** la proportion pondérale d'Ester de Sorbitan et d'Acide(s) Gras (ESAG) : solvant (S) est comprise entre 20 : 80 et 80 : 20, de préférence est comprise entre 30 : 70 et 70 : 30, et plus préférentiellement encore correspond à 40 : 60 ou à 60 : 40.

9. Milieu selon l'une quelconque des revendications 1 à 8, **caractérisé en ce que** la concentration en [substrat] dans le milieu, est définie comme suit (en mg/l) :
| | |
|---|---|
| | 1 ≤ [substrat] ≤ 2000, |
| de préférence | 5 ≤ [substrat] ≤ 1500, |
| et plus préférentiellement encore | 25 ≤ [substrat] ≤ 1000. |

10. Milieu selon l'une quelconque des revendications 1 à 9, **caractérisé en ce que** le milieu de culture qu'il comprend est sélectionné dans le groupe comprenant:
- les milieux sélectifs de type Mac Conkey, Columbia ANC, PALCAM, Sabouraud gentamycine-chloramphénicol, de préférence le milieu Mac Conkey,
- les milieux non sélectifs de type Columbia +/- sang, Trypcase Soja, Gélose nutritive, Sabouraud, de préférence le milieu Columbia.

11. Milieu selon l'une quelconque des revendications 1 à 10, **caractérisé en ce que** les microorganismes à détecter/identifier font partie des microorganismes d'intérêt médical ou industriel, et notamment ceux du genre *Salmonella, Pseudomonas, Listeria, Siaphylacoccus*, *Enterococcus* ou *Candida.*

12. Procédé d'obtention du milieu selon l'une quelconque des revendications 1 à 11 **caractérisé en ce qu'**il consiste essentiellement :
- à préparer au moins une solution-mère du substrat d'estérases et/ou d'osidases et/ou de peptidases et/ou de sulfatases et/ou de phosphatases, chromogène ou fluorogène, dans le solvant S et d'au moins un ESAG, AG ou ESAG/AG,
- à ajouter cette solution et d'éventuels autres additifs dans le milieu de culture,
- et à homogénéiser l'ensemble.

13. Procédé détection/identification de souches à activité estérase et/ou osidase et/ou peptidase et/ou sulfatase et/ou phosphatase **caractérisé en ce qu'**il consiste :
- à ensemencer le milieu selon l'une quelconque des revendications 1 à 11 ou le milieu obtenu par le procédé selon la revendication 12, avec les bactéries à analyser;
- à incuber le milieu ensemencé dans des conditions appropriées,
- et à lire et interpréter les colorations autour des colonies, lesquelles colorations révélant l'hydrolyse du substrat par les bactéries.

14. Utilisation d'Ester(s) de Sorbitan et d'Acide(s) Gras (ESAG), d'Acidc(s) Gras (AG) ou de ESAG/AG, à titre d'agent stabilisant-émulsifiant de milieu (liquide ou gel) de détection/identification de microorganismes, notamment de bactéries ou de levures, à activité enzymatique choisie parmi les activités estérases et/ou osidases et/ou peptidases et/ou sulfatases et/ou phosphatases, ce milieu comprenant notamment un milieu de culture et au moins un substrat d'estérases et/ou d'osidases et/ou de peptidases et/ou de sulfatases et/ou de phosphatases, chromogène ou fluorogène, à l'exclusion des substrats comprenant un carboxylate de 4-[2-(4-octanoyloxy-3,5-diméthoxyphényl)-vinyl]-quinolinium-1-(propan-3-yle) et un anion, éventuellement en association avec au moins un solvant S du substrat d'estérases et/ou d'osidases et/ou de peptidases et/ou de sulfatases et/ou de phosphatases, chromogène ou fluorogène, présent dans le milieu; la concentration en [ESAG] dans le milieu étant définie comme suit (en % en poids): 0,5 ≤ [ESAG] ≤ 5, de préférence 1,5 ≤ [ESAG] ≤ 3,5.

## Claims

1. Medium for the detection/identification of microorganisms, especially bacteria and/or yeasts, with an enzymatic activity selected from esterase and/or osidase and/or peptidase and/or sulfatase and/or phosphatase activities, said medium being of the type comprising especially a reaction medium and at least one chromogenic or fluorogenic esterase, osidase and/or peptidase and/or sulfatase and/or phosphatase substrate, with the exclusion of substrates comprising a 4-[2-(4-octanoyloxy-3,5-dimethoxyphenyl)vinyl]quinolinium-1-(propan-3-ylcarboxylic acid) cation and an anion, this detection/identification being based essentially on revealing the presence of the esterase and/or osidase and/or peptidase and/or sulfatase and/or phosphatase activity, ... said medium:
* is in a ready-to-use liquid or gel form that is stable on storage, i.e. the intensity of the coloration or fluorescence is maintained at a maximum level for at least several weeks; and
* contains:
- at least one fatty acid sorbitan ester (FASE) or at least one fatty acid (FA) or an FASE/FA mixture as an emulsifying stabilizer,
- the concentration of (FASE) in the medium is defined as follows (in % by weight): 0.5 ≤ [FASE] ≤ 5, preferably 1.5 ≤ [FASE] ≤ 3.5, and
- optionally at least one solvent (S).

2. Medium according to claim 1, **characterized in that** it is obtained by mixing at least one stock solution of substrate in the solvent S, and FASE, FA or an FASE/FA mixture, with at least some of the constituents of the culture medium.

3. Medium according to claim 1 or 2, **characterized in that** the FASE is selected from the group comprising:
• polyethoxylated sorbitan monolaurate containing 20 units of ethylene oxide (EO) - TWEEN® 20 - ;
• polyethoxylated sorbitan monopalmitate (20 EO) - TWEEN® 40 -;
• polyethoxylated sorbitan monostearate (20 EO) - TWEEN® 60 - ;
• polyethoxylated sorbitan tristearate (20 EO) - TWEEN® 65 - ;
• polyethoxylated sorbitan monooleate (20 EO) - TWEEN® 80 - ;
• polyethoxylated sorbitan sesquioleate (20 EO) - TWEEN® 83 - ;
• polyethoxylated sorbitan trioleate (20 EO) - TWEEN® 85 - ; and
• mixtures thereof.

4. Medium according to claim 1 or 2, **characterized in that** the FA is selected from the group comprising C4-C20 saturated or unsaturated fatty acids, preferably C6-C11 saturated or unsaturated fatty acids and particularly preferably C8-C9 fatty acids, and mixtures thereof.

5. Medium according to any one of claims 1 to 4, **characterized in that** it also comprises at least one anionic surfactant, preferably 7-ethyl-2-methyl-4-undecyl hydrogen sulfate or its salts, more particularly its sodium salts (TERGITOL-4® ).

6. Medium according to any one of claims 1 to 5, **characterized in that** the chromogenic or fluorogenic substrate consists of a target part for the enzyme and a chromophoric or fluorophoric part, the target part being selected from the group comprising the following in particular:
- glycosides consisting of mono-, di- and/or polysaccharide units linked in the α- or β-position to the hydroxyl group of the fluorophoric or chromophoric part;
- α-amino acids or peptides;
- organic acids such as -O-CO(CH₂)ₙ-CH₃, where n is between 0 and 20; and
- a sulfate, a phosphate, a pyrosulfate, a pyrophosphate and a phosphodiester,
and the chromophoric or fluorophoric part being selected from the group comprising the following in particular:
- quinones/anthraquinones and derivatives, especially dihydroxyanthraquinone (alizarin);
- amino- and hydroxycoumarins and derivatives;
- fluoresceins and derivatives; and
- indoxyls and derivatives.

7. Medium according to any one of claims 1 to 6, **characterized in that** the solvent S is selected from the group comprising:
- alcohols, preferably methanol, ethanol and methoxyethanol;
- amides, preferably dimethylformamide (DMF);
- sulfur-containing solvents, preferably dimethyl sulfoxide (DMSO);
- aqueous solvents, preferably water and buffered water; and
- mixtures thereof.

8. Medium according to any one of claims 1 to 7, **characterized in that** the proportion by weight of fatty acid sorbitan ester(s) (FASE) to solvent (S) is between 20:80 and 80:20, preferably between 30:70 and 70:30 and particularly preferably 40:60 or 60:40.

9. Medium according to any one of cl'aims 1 to 8, **characterized in that** the concentration of substrate in the medium is defined as follows (in mg/l):
| | |
|---|---|
| | 1 ≤ [substrate] ≤ 2000, |
| preferably | 5 ≤ [substrate] ≤ 1500, |
| and particularly preferably | 25 ≤ [substrate] ≤ 1000. |

10. Medium according to any one of claims 1 to 9, **characterized in that** the culture medium it contains is selected from the group comprising:
- selective media of the type comprising MacConkey, Columbia ANC, PALCAM and Sabouraud gentamycin-chloramphenicol media, preferably MacConkey medium; and
- non-selective media of the type comprising Columbia +/- blood, trypcase soya, nutrient gelose and Sabouraud media, preferably Columbia medium.

11. Medium according to any one of claims 1 to 10, **characterized in that** the microorganisms to be detected/identified are microorganisms of medical or industrial interest, especially those of the genus *Salmonella, Pseudomonas, Listeria, Staphylococcus, Enterococcus* or *Candida.*

12. Method of obtaining the medium according to any one of claims 1 to 11, **characterized in that** it consists essentially in:
- preparing at least one stock solution of the chromogenic or fluorogenic esterase and/or osidase and/or peptidase and/or sulfatase and/or phosphatase substrate in the solvent S and of at least one FASE, FA or FASE/FA,
- adding this solution and any other additives to the culture medium, and
- homogenizing the whole.

13. Method for the detection/identification of strains with esterase and/or osidase and/or peptidase and/or sulfatase and/or phosphatase activity, **characterized in that** it consists in:
- inoculating the medium according to any one of claims 1 to 11, or the medium obtained by the method according to claim 12, with the bacteria to be analyzed;
- incubating the inoculated medium under appropriate conditions; and
- reading and interpreting the colorations around the colonies, these colorations revealing hydrolysis of the substrate by the bacteria.

14. Use of fatty acid sorbitan ester(s) (FASE), fatty acid(s) (FA) or FASE/FA as an emulsifying stabilizer for a medium (liquid or gel) for the detection/identification of microorganisms, especially bacteria or yeasts, with an enzymatic activity selected from esterase and/or osidase and/or peptidase and/or sulfatase and/or phosphatase activities, this medium comprising especially a culture medium and at least one chromogenic or fluorogenic esterase and/or osidase and/or peptidase and/or sulfatase and/or phosphatase substrate, with the exclusion of substrates comprising a 4-[2-(4-octanoyloxy-3,5-dimethoxyphenyl)vinyl]quinolinium-1-(propan-3-yl)carboxylate and an anion, optionally in association with at least one solvent S for the chromogenic or fluorogenic esterase and/or osidase and/or peptidase and/or sulfatase and/or phosphatase substrate present in the medium, the concentration of (FASE) in the medium being defined as follows (in % by weight): 0.5 ≤ [FASE] ≤ 5, preferably 1.5 ≤ [FASE] ≤ 3.5.

## Patentansprüche

1. Medium zum Nachweisen/Identifizieren von Mikroorganismen und insbesondere von Bakterien und/oder Hefen mit enzymatischer Aktivität, ausgewählt aus Esterase- und/oder Osidase- und/oder Peptidase- und/oder Sulfatase- und/oder Phosphatase-Aktivität, welches insbesondere ein Reaktionsmedium und wenigstens ein chromogenes oder fluorogenes Esterase-, Osidase- und/oder Peptidase- und/oder Sulfatase- und/oder Phosphatase-Substrat umfasst, wobei Substrate ausgenommen sind, die ein Kation von 4-[2-(4-Oktanoyloxy-3,5-dimethoxyphenyl)-vinyl]chinolinium-1-(propan-3-yl-carbonsäure) und ein Anion umfassen,
wobei dieses Nachweisen/Identifizieren im Wesentlichen darauf beruht, die Esterase- und/oder Osidase- und/oder Peptidase- und/oder Sulfatase-
und/oder Phosphatase-Aktivität aufzuzeigen,
wobei das Medium
• in gebrauchsfertiger und lagerungsbeständiger flüssiger Form oder Gelform vorliegt, d.h. die Intensität der Färbung oder der Fluoreszenz wird für wenigstens mehrere Wochen auf einem Maximalwert gehalten,
• umfasst:
- wenigstens einen Fettsäuresorbitanester (ESAG) oder wenigstens eine Fettsäure (AG) oder ein ESAG/AG-Gemisch als emulgierendes Stabilisierungsmittel,
- die Konzentration an [ESAG] in dem Medium ist wie folgt definiert (in Gew.-%): 0,5 ≤ [ESAG] ≤ 5, vorzugsweise 1,5 ≤ [ESAG] ≤ 3,5;
- und gegebenenfalls wenigstens ein Lösungsmittel (S).

2. Medium nach Anspruch 1, **dadurch gekennzeichnet, dass** es durch Vermischen von wenigstens einer Substrat-Stammlösung in dem Lösungsmittel S und von ESAG, AG oder ESAG/AG-Gemisch mit wenigstens einem Teil der Bestandteile des Kulturmediums erhalten wird.

3. Medium nach Anspruch 1 oder 2, **dadurch gekennzeichnet, dass** der ESAG ausgewählt ist aus der Gruppe umfassend:
• Polyoxyethylen-Sorbitan-Monolaurat, welches 20 Ethylenoxid-Gruppen (EO) umfasst, - TWEEN® 20 -;
• Polyoxyethylen-Sorbitan-Monopalmitat (20 EO), - TWEEN® 40 -;
• Polyoxyethylen-Sorbitan-Monostearat (20 EO), - TWEEN® 60 -;
• Polyoxyethylen-Sorbitan-Tristearat (20 EO), - TWEEN® 65 -;
• Polyoxyethylen-Sorbitan-Monooleat (20 EO), - TWEEN® 80 -;
• Polyoxyethylen-Sorbitan-Sesquioleat (20 EO), - TWEEN® 83 -;
• Polyoxyethylen-Sorbitan-Trioleat (20 EO), - TWEEN® 85 -;
• und deren Gemische.

4. Medium nach Anspruch 1 oder 2, **dadurch gekennzeichnet, dass** die AG ausgewählt ist aus der Gruppe, umfassend gesättigte oder ungesättigte Fettsäuren mit C4-C20, bevorzugt gesättigte oder ungesättigte Fettsäuren mit C6-C11 und weiter bevorzugt Fettsäuren mit C8-C9 und deren Gemische.

5. Medium nach einem der Ansprüche 1 bis 4, **dadurch gekennzeichnet, dass** es weiter wenigstens ein anionisches oberflächenaktives Mittel umfasst, vorzugsweise 7-Ethyl-2-methyl-4-undekyl-hydrogensulfat oder dessen Salze und insbesondere dessen Natriumsalze (TERGITOL-4 ®).

6. Medium nach einem der Ansprüche 1 bis 5, **dadurch gekennzeichnet, dass** das chromogene oder fluorogene Substrat aus einem Ziel-Teil für das Enzym und einem chromophoren oder fluorophoren Teil gebildet ist, wobei der Ziel-Teil ausgewählt ist aus der Gruppe umfassend insbesondere:
- Glycoside, die aus Mono-, Di- und/oder Polysacchariden gebildet sind, die in der α- oder β-Position an die Hydroxylfunktion des chromophoren oder fluorophoren Teils gebunden sind;
- α-Aminosäuren oder Peptide;
- organische Säuren wie beispielsweise -O-CO(CH₂)ₙ-CH₃, mit n zwischen einschließlich 0 und 20;
- ein Sulfat, ein Phosphat, ein Pyrosulfat, ein Pyrophosphat oder einen Phosphodiester;
wobei der chromophore oder fluorophore Teil ausgewählt ist aus der Gruppe umfassend insbesondere:
- Chinone/Anthrachinone und Derivate, insbesondere Dihydroxyanthrachinon (Alizarin);
- Amino- oder Hydroxy-Cumarine und Derivate;
- Fluoreszeine und Derivate;
- Indoxyle und Derivate.

7. Medium nach einem der Ansprüche 1 bis 6, **dadurch gekennzeichnet, dass** das Lösungsmittel S ausgewählt ist aus der Gruppe umfassend:
- Alkohole, vorzugsweise Methanol, Ethanol, Methoxyethanol;
- Amide, vorzugsweise Dimethylformamid (DMF);
- schwefelhaltige Lösungsmittel, vorzugsweise Dimethylsulfoxid (DMSO);
- wässrige Lösungsmittel, vorzugsweise Wasser, gepuffertes Wasser;
- und deren Gemische.

8. Medium nach einem der Ansprüche 1 bis 7, **dadurch gekennzeichnet, dass** das Gewichtsverhältnis von Fettsäuresorbitanester(n) (ESAG) zu Lösungsmittel (S) zwischen einschließlich 20 : 80 und 80 : 20 liegt, vorzugsweise zwischen einschließlich 30 : 70 und 70 : 30 und noch weiter bevorzugt 40 : 60 oder 60 : 40 entspricht.

9. Medium nach einem der Ansprüche 1 bis 8, **dadurch gekennzeichnet, dass** die [Substrat]-Konzentration in dem Medium wie folgt definiert ist (in mg/l):
| | |
|---|---|
| | 1 ≤ [Substrat] ≤ 2000, |
| bevorzugt | 5 ≤ [Substrat] ≤ 1500, |
| noch weiter bevorzugt | 25 ≤ [Substrat] ≤ 1000. |

10. Medium nach einem der Ansprüche 1 bis 9, **dadurch gekennzeichnet, dass** das Kulturmedium, welches darin enthalten ist, ausgewählt ist aus der Gruppe umfassend:
- Selektionsmedien vom Typ Mac Conkey, Columbia ANC, PALCAM, Sabouraud Gentamycin-Chloramphenicol, vorzugsweise Mac Coney Medium,
- nicht-selektive Medien vom Typ Columbia +/- Blut, Trypcase Soja, Nähr-Gelose, Sabouraud, vorzugsweise Columbia Medium.

11. Medium nach einem der Ansprüche 1 bis 10, **dadurch gekennzeichnet, dass** die Mikroorganismen, die nachgewiesen/detektiert werden sollen, Mikroorganismen von medizinischem oder industriellem Interesse, und insbesondere von der Gattung *Salmonella, Pseudomonas, Listeria, Staphylococcus, Enterococcus* oder *Candida* sind.

12. Verfahren zum Erhalten eines Mediums nach einem der Ansprüche 1 bis 11, **dadurch gekennzeichnet, dass** es insbesondere daraus besteht:
- wenigstens eine Substrat-Stammlösung von chromogenen oder fluorogenen Esterasen und/oder Osidasen und/oder Peptidasen und/oder Sulfatasen und/oder Phosphatasen in dem Lösungsmittel S und wenigstens einem ESAG, einer AG oder einem ESAG/AG herzustellen,
- diese Lösung und gegebenenfalls weitere Zusatzstoffe zu dem Kulturmedium zuzugeben
- und das Ganze zu homogenisieren.

13. Verfahren zum Nachweisen/Identifizieren von Stämmen mit Esterase- und/oder Osidase- und/oder Peptidase- und/oder Sulfatase- und/oder Phosphatase-Aktivität, **dadurch gekennzeichnet, dass** es daraus besteht:
- das Medium nach einem der Ansprüche 1 bis 11 oder das durch das Verfahren nach Anspruch 12 erhaltene Medium mit den zu analysierenden Bakterien zu beimpfen;
- das beimpfte Medium unter geeigneten Bedingungen zu inkubieren,
- und die Färbungen in der Umgebung der Kolonien zu lesen und zu interpretieren, wobei die Färbungen die Hydrolyse des Substrats durch die Bakterien anzeigen.

14. Verwendung von Fettsäuresorbitanester(n) (ESAG), Fettsäure(n) (AG) oder ESAG/AG als emulgierendes Stabilisierungsmittel eines (flüssigen oder gelförmigen) Mediums zum Nachweisen/Identifizieren von Mikroorganismen, insbesondere Bakterien oder Hefen mit enzymatischer Aktivität, ausgewählt aus Esterase- und/oder Osidase- und/oder Peptidase- und/oder Sulfatase- und/oder Phosphatase-Aktivität, wobei dieses Medium insbesondere ein Kulturmedium und wenigstens ein chromogenes oder fluorogene Esterase- und/oder Osidase- und/oder Peptidase- und/oder Sulfatase- und/oder Phosphatase-Substrat umfasst, wobei Substrate ausgenommen sind, die ein Carboxylat von 4-[2-(4-Oktanoyloxy-3,5-dimethoxyphenyl)-vinyl]chinolinium-1-(propan-3-yl) und ein Anion umfassen, gegebenenfalls zusammen mit wenigstens einem Lösungsmittel S für das in dem Medium vorhandene chromogene oder fluorogene Esterase- und/oder Osidase- und/oder Peptidase- und/oder Sulfatase- und/oder Phosphatase-Substrat, wobei die [ESAG]-Konzentration in dem Medium wie folgt definiert ist (in Gew.-%): 0,5 ≤ [ESAG] ≤ 5, vorzugsweise 1,5 ≤ [ESAG] ≤ 3,5.
